(19) Europäisches Patentamt
European Patent Office
Office européen des brevets

(11) EP 0 820 439 B1

(12) FASCICULE DE BREVET EUROPEEN

(45) Date de publication et mention de la délivrance du brevet: **03.05.2000 Bulletin 2000/18**

(21) Numéro de dépôt: **97904485.6**

(22) Date de dépôt: **04.02.1997**

(51) Int Cl.$^7$: **C07C 327/32**, C07B 53/00 // C07C33/26, C07C69/16, C07C59/48

(86) Numéro de dépôt international: **PCT/FR97/00218**

(87) Numéro de publication internationale: **WO 97/29086 (14.08.1997 Gazette 1997/35)**

# (54) PROCEDE DE SYNTHESE ASYMETRIQUE DES DERIVES S-ACYLES DE L'ACIDE 2-MERCAPTOMETHYL 3-PHENYL PROPANOIQUE, APPLICATION A LA SYNTHESE DE DERIVES N-(MERCAPTOACYL) AMINO-ACIDES

VERFAHREN ZUR ASYMMETRISCHEN SYNTHESE VON S-ACYLIERTEN DERIVATEN VON 2-MERCAPTOMETHYL-3-PHENYLPROPIONSÄURE UND DEREN VERWENDUNG ZUR SYNTHESE VON N-MERCAPTOACYL AMINOSÄURE-DERIVATEN

METHOD FOR THE ASYMMETRICAL SYNTHESIS OF S-ACYLATED DERIVATIVES OF 2-MERCAPTOMETHYL 3-PHENYL PROPANOIC ACID, AND USE THEREOF FOR SYNTHESISING N-(MERCAPTOACYL) AMINO ACID DERIVATIVES

(84) Etats contractants désignés:
**AT BE CH DE DK ES FI FR GB GR IE IT LI LU MC NL PT SE**

(30) Priorité: **05.02.1996 FR 9601360**

(43) Date de publication de la demande: **28.01.1998 Bulletin 1998/05**

(73) Titulaire: **SOCIETE CIVILE BIOPROJET**
**F-75003 Paris (FR)**

(72) Inventeurs:
- **DANVY, Denis**
  **F-76190 Yvetot (FR)**
- **MONTEIL, Thierry**
  **F-76130 Mont-Saint-Aignan (FR)**
- **DUHAMEL, Pierre**
  **F-76130 Mont-Saint-Aignan (FR)**
- **DUHAMEL, Lucette**
  **F-76130 Mont-Saint-Aignan (FR)**
- **LECOMTE, Jeanne-Marie**
  **F-75003 Paris (FR)**
- **SCHWARTZ, Jean-Charles**
  **F-75014 Paris (FR)**
- **NOEL-LEFEBRVRE, Nadine**
  **78760 Jouars Pontchartrin (FR)**
- **GROS, Claude**
  **75015 Paris (FR)**
- **PLAQUEVENT, Jean-Christophe**
  **76250 Deville-Les-Rouens (FR)**

(74) Mandataire: **Bernasconi, Jean et al**
**c/o Cabinet Lavoix,**
**2, Place d'Estienne d'Orves**
**75441 Paris Cédex (FR)**

(56) Documents cités:
**EP-A- 0 136 883**
**EP-A- 0 377 139**
**EP-A- 0 501 870**
**FR-A- 2 623 498**

- **LIEBIGS ANN. CHEM., no. 10, 1989, pages 957-962, XP000611532 K. MORI ET AL:**
- **TETRAHEDRON LETT., vol. 31, no. 11, 1990, pages 1601-1604, XP002020256 S. ATSUUMI ET AL: cité dans la demande**
- **RECUEIL DE TRAVAUX CHIMIQUES DES PAYS-BAS, vol. 106, no. 10, 1987, pages 539-542, XP000196631 B. STRIJTVEEN ET AL:**
- **TETRAHEDRON: ASYMMETRY, vol. 4, no. 9, 1993, pages 1995-2000, XP000611535 G. CARON ET AL:**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne un procédé de synthèse énantiosélectif des dérivés S-acylés de l'acide 2-mercaptométhyl 3-phényl propanoïque et leur utilisation dans la synthèse asymétrique de dérivés N-(mercaptoacyl) amino-acides. Plus spécifiquement, la présente invention concerne un nouveau procédé de synthèse de dérivés optiquement purs de formule générale (I) :

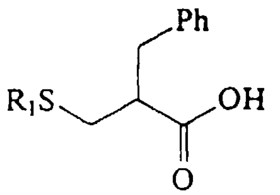 (I)

dans laquelle :
$R_1$ représente un radical acyle aliphatique linéaire ou ramifié ou un radical acyle aromatique.
Les dérivés de formule (I) obtenus selon le procédé de l'invention sont utiles en particulier pour la synthèse de dérivés N-(mercaptoacyl)amino-acides optiquement actifs de formule (II) :

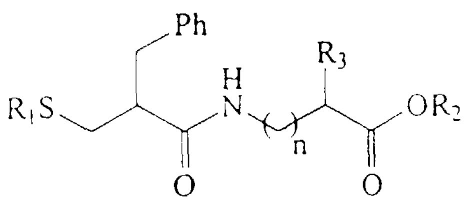 (II)

dans laquelle :

$R_1$ représente un atome d'hydrogène, un radical acyle aliphatique linéaire ou ramifié ou un radical acyle aromatique,
$R_2$ représente un atome d'hydrogène, un radical alkyle inférieur, un radical phényle ou un groupe phénylalkylène inférieur ;
$R_3$ représente un atome d'hydrogène ; un groupe alkyle inférieur ; un groupe hydroxyalkylène inférieur ; un groupe phényle ; un groupe phénylakylène inférieur ; un groupe hydroxyphénylalkylène inférieur ; un groupe aminoalkylène inférieur ; un groupe guanidinoalkylène inférieur ; un groupe mercaptoalkylène inférieur ; un groupe alkyle inférieur thioalkylène inférieur ; un groupe imidazolylalkylène inférieur ; un groupe indolylalkylène inférieur ; un groupe carbamylalkylène inférieur ; un groupe carboxyalkylène inférieur ;
n varie de 0 à 10.

Par groupe alkyle inférieur, on entend des groupes alkyles à chaîne linéaire ou ramifiée contenant de 1 à 6 atomes de carbone et, de préférence, 1 à 4 atomes de carbone.
Par groupe alkykène inférieur, on entend des groupes alkylènes contenant de 1 à 6 atomes de carbone et, de préférence, 1 à 4 atomes de carbone.
Les composés de formule (II) préférés sont les composés répondant aux formules (III) et (IV) suivantes :

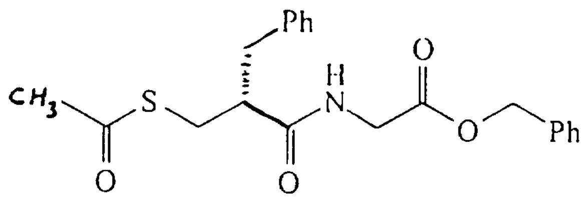 (III)

ou le N-(R)-[2-acétylthiométhyl-1-oxo-3 phényl-propyl]-glycinate de benzyle ;

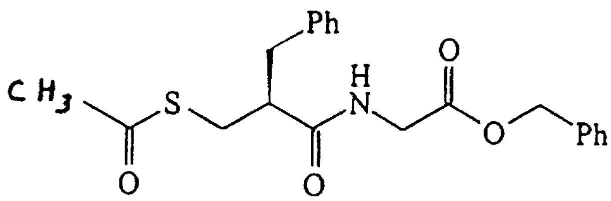 (IV)

ou le N-(S)-[2-acétylthiométhyl-1-oxo-3 phényl-propyl]-glycinate de benzyle,
pour lesquels le procédé de la présente invention peut être plus particulièrement appliqué.

Les composés de formule (II) possèdent des propriétés pharmacologiques intéressantes. Ils exercent notamment une activité inhibitrice sur certaines enzymes, comme l'endopeptidase neutre (EC 3.4.24.11) et l'enzyme de conversion de l'angiotensine (EC 3.4.15.1). L'administration des composés de formule (II) permet donc de réduire ou de supprimer l'activité de ces enzymes, responsables respectivement de l'inactivation des enképhalines, du facteur atrial natriurétique, et de la transformation de l'angiotensine I en angiotensine II. En thérapeutique, ces composés exercent des activités antisécrétoires, intestinales ou antihypertensives et sont utilisés dans le traitement de l'insuffisance cardiaque chronique. De plus, de tels composés peuvent sont utilisés dans le traitement de l'insuffisance cardiaque chronique. De plus, de tels composés peuvent être également utilisés dans le traitement de l'ostéoporose (WO. 94/21242).

Les composés de formule (II) et, plus particulièrement les composés de formules (III) et (IV), leur préparation et leur utilisation en thérapeutique ont été décrits dans le brevet français n° 2.623.498.

On peut utiliser les composés de formule (I) pour la préparation des composés de formule (II).

Les composés de formule (I) comprennent un carbone, dit asymétrique, pouvant avoir deux configurations R ou S. Ils existent donc sous forme de deux énantiomères, que l'on peut désigner par (I R) et (I S), selon la configuration de ce carbone asymétrique :

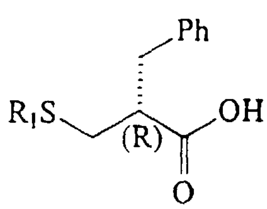

(IR)

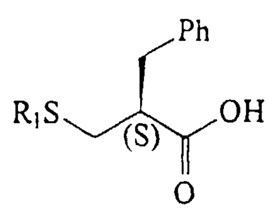

(IS)

Aucun procédé de synthèse asymétrique des dérivés de formule (I) n'a été décrit jusqu'à présent.

On connaît le brevet européen n° EP 318 377 dans lequel les deux énantiomères (I R) et (I S) de l'acide de formule générale (I) sont obtenus par séparation du mélange racémique à l'aide d'une amine chirale. Toutefois, cette méthode de dédoublement ne permet d'obtenir au maximum que cinquante (50) pour cent de l'énantiomère désiré.

La présente invention a pour objectif de fournir un procédé de préparation des dérivés S-acylés de l'acide 2-mercaptométhyl 3-phényl propanoique, sous forme optiquement pure.

Un autre objectif de l'invention est aussi de fournir un tel procédé ne présentant pas l'inconvénient d'une séparation optique telle que précitée.

L'invention a encore pour objectif de fournir un procédé de préparation de dérivés N-(mercaptoacyl) amino-acides optiquement actifs à partir des dérivés S-acylés de l'acide 2-mercaptométhyl 3-phényl propanoïque.

A cette fin, l'invention a pour objet un procédé de synthèse asymétrique des dérivés S-acylés de l'acide 2-mercaptométhyl 3-phényl propanoïque de formule (I) :

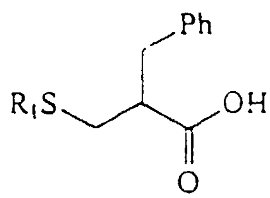

(I)

dans laquelle
$R_1$ représente un radical acyle aliphatique linéaire ou ramifié ou un radical acyle aromatique, caractérisé en ce qu'il comprend les étapes consistant à :

a) préparer le diol de formule (VI) :

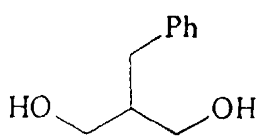

(VI)

par réduction d'un ester malonique de formule (V) :

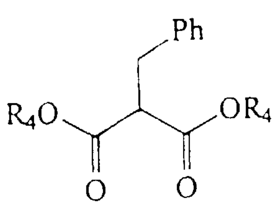

(V)

dans laquelle $R_4$ représente une chaîne alkyle contenant de 1 à 4 atomes de carbone, en présence d'un hydrure ;
b) préparer le monoacétate (VII) respectivement de configuration (R) et de configuration (S), de formules (VII R) et (VII S) :

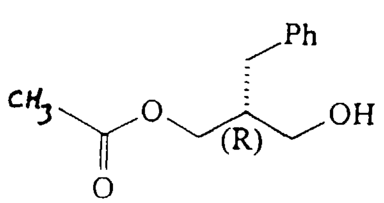

(VIIR)

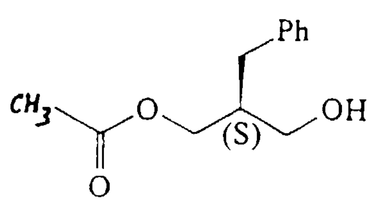

(VIIS)

c) soumettre les monoacétates de formule (VII R) ou (VII S) à une oxydation pour former les acides de formule (IX S) ou (IX R) :

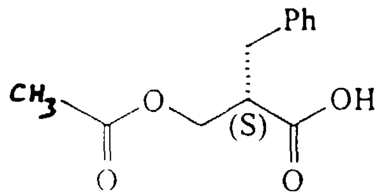

(IXS)

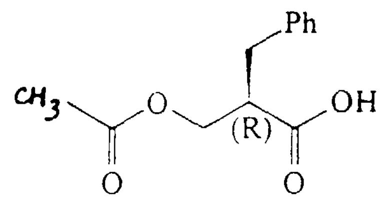

(IXR)

d) saponifier les composés de formule (IX S) ou (IX R), en présence d'une solution aqueuse basique pour former les hydroxyacides de formule (X S) ou (X R) :

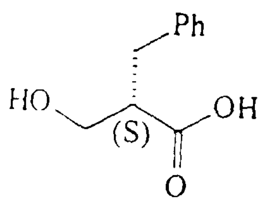

(XS)

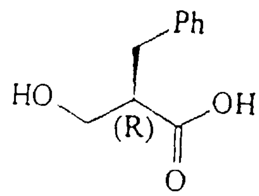

(XR)

e) thioacyler les hydroxyacides de formule (X S) ou (X R) avec un mercaptoacide de formule (XI) :

R1 S H (XI)

dans laquelle $R_1$ représente un radical acyle aliphatique linéaire ou ramifié ou un radical acyle aromatique, selon une réaction de type Mitsunobu en présence d'un complexe d'azodicarboxylate d'alkyle/triphénylphosphine pour conduire respectivement aux acides de formule (I R) ou (I S) désirés:

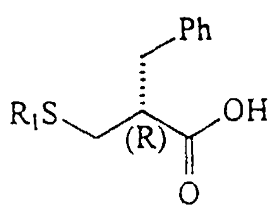

(IR)

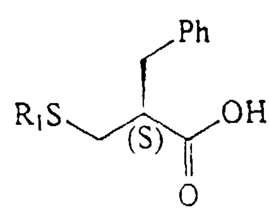

(IS)

Elle a encore pour objet un procédé de préparation de dérivés N-(mercaptoacyl)amino-acides optiquement actifs de formule (II) :

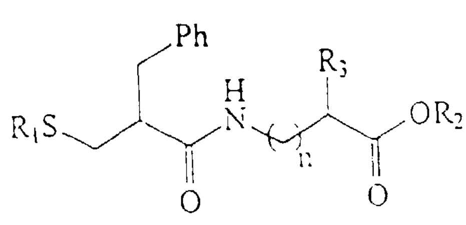

(II)

dans laquelle

$R_1$ représente un atome d'hydrogène, un radical acyle aliphatique linéaire ou ramifié ou un radical acyle aromatique,
$R_2$ représente un radical alkyle inférieur, un radical phényle ou un groupe phénylalkylène inférieur,
$R_3$ représente un atome d'hydrogène ; un groupe alkyle inférieur ; un groupe hydroxylalkylène inférieur ; un groupe phényle ; un groupe phénylalkylène inférieur ; un groupe hydroxyphénylalkylène inférieur ; un groupe aminoalkylène inférieur ; un groupe guanidinoalkylène inférieur ; un groupe mercaptoalkylène inférieur ; un groupe alkyle inférieur thioalkylène inférieur ; un groupe imidazolyalkylène inférieur ; un groupe indolylalkylène inférieur ; un groupe carbamylalkylène inférieur ; un groupe carboxyalkylène inférieur ;
n varie de 0 à 10 ;

caractérisé en ce qu'il comprend l'utilisation des dérivés S-acylés de l'acide 2-mercaptométhyl 3-phényl propanoique de formule (I) sous forme optiquement pure tels qu'obtenus selon le procédé précité.

Enfin, l'invention a aussi pour objet l'utilisation des dérivés S-acylés de l'acide 2-mercapto-méthyl-3-phényl propanoïque de formule (I) sous forme optiquement pure tels qu'obtenus par le procédé précité, pour la synthèse de dérivés N-(mercaptoacyl) amino-acides optiquement actifs de formule (II) donnée ci-dessus.

La demanderesse a maintenant trouvé un procédé de synthèse des dérivés de formule (I) sous forme optiquement pure, qui ne présente pas l'inconvénient d'une séparation optique.

Selon une caractéristique essentielle du procédé conforme à l'invention, celui-ci utilise comme matière première les esters d'acides benzylmaloniques de formule (V) :

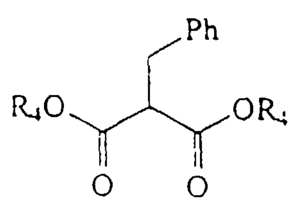

(V)

dans laquelle $R_4$ représente une chaîne alkyle contenant de un à quatre atomes de carbone.

Les esters maloniques de formule (V) sont ensuite réduits à l'aide d'un hydrure, tel que l'aluminohydrure de lithium, pour conduire au diol de formule (VI) :

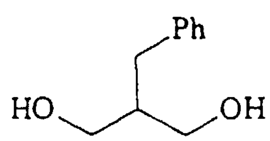

(VI)

On peut également utiliser le borohydrure de sodium.

Le diol de formule (VI) est ensuite monoacétylé à l'aide d'une enzyme, telle que la lipase PS (Amano) ou la lipase issue de Pseudomonas fluorescens (Fluka), dans l'acétate de vinyle pour conduire au monoacétate (VII R) de configuration (R) :

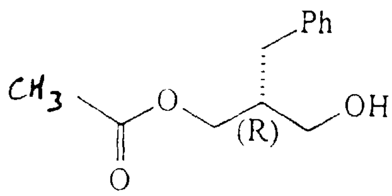

(VIIR)

Pour l'obtention du monoacétate de configuration (S), le diol de formule (VI) est diacétylé soit par action de l'anhydride acétique en présence d'un catalyseur tel que, par exemple, le mélange 4-diméthylaminopyridine/triéthylamine ou l'acide sulfurique, soit à l'aide d'une enzyme telle que la Novozym 435 (Novo Nordisk) dans l'acétate de vinyle, pour conduire au diacétate de formule (VIII) :

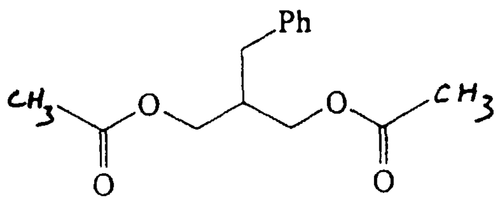

(VIII)

Le diacétate de formule (VIII) est ensuite mono-hydrolysé à l'aide d'une enzyme, telle que la lipase PS (Amano) ou la lipase issue de Pseudomonas fluorescens (Fluka), en milieu aqueux, pour conduire au monoacétate (VII S) de configuration (S) :

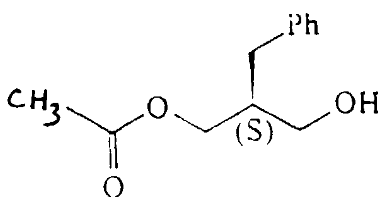

(VIIS)

Le monoacétate de formule (VII R) ou (VII S) est ensuite oxydé à l'aide d'un oxydant, tel que le réactif de Jones ($CrO_3$-$H_2SO_4$), dans un solvant, tel que l'acétone, pour conduire à l'acide (IX S) de configuration (S) ou à l'acide de formule (IX R) de configuration (R) :

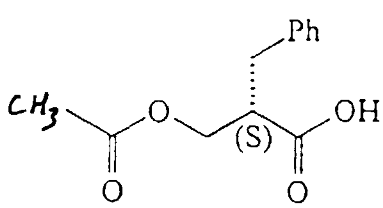

(IXS)

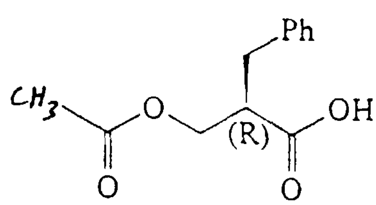

(IXR)

On peut également utiliser, comme oxydant, le permanganate de potassium ou l'acide nitrique.

Le composé de formule (IX S) ou (IX R) est ensuite saponifié par une solution aqueuse basique, telle qu'une solution aqueuse de soude ou une solution aqueuse de lithine, pour conduire à l'hydroxyacide (X S) de configuration (S) ou à l'hydroxyacide (X R) de configuration (R) :

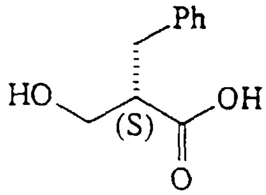
(XS)

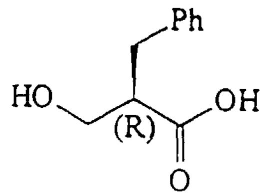
(XR)

La préparation énantiosélective des monoacétates (VII S) et (VII R) puis d'acides 3-hydroxy-propioniques 2-substitués à l'aide de Lipase P est connue des publications K. TSUJI et al., Tett. Lett., 30 (45), 6189-6192 (1989) et S. ATSUUMI et al., Tett. Lett., 31 (11), 1601-1604 (1990), ces composés servant d'intermédiaires dans la synthèse d'inhibiteurs de la rénine à partir d'acides 3-alkyl (ou aryl)sulfonylpropioniques 2-substitués.

L'hydroxyacide de formule (X S) ou (X R) est ensuite thioacylé selon une réaction de type Mitsunobu (O. MITSUNOBU, Synthesis, pp. 1-27, 1991) en présence d'un complexe d'azodicarboxylate d'alkyle/triphényl-phosphine et d'un mercaptoacide de formule (XI) :

$$R1\ S\ H \qquad (XI)$$

dans laquelle $R_1$ a la même signification que dans la formule (I), pour conduire aux acides de formule (I R) de configuration (R), ou (I S) de configuration (S).

L'azodicarboxylate d'alkyle utilisé pour la réaction de Mitsunobu est, de préférence, choisi parmi l'azodicarboxylate de diisopropyle ou l'azodicarboxylate de diéthyle.

Le mercaptoacide (XI) utilisé pour la réaction de Mitsunobu est, de préférence, choisi parmi l'acide thioacétique ou l'acide thiobenzoïque.

Le procédé conforme à l'invention est plus particulièrement adapté à la préparation de l'acide (R)-2-acétylthiométhyl-3-phényl propanoïque de formule (Ia R) et de l'acide (S)-2-acétylthiométhyl-3-phényl propanoïque de formule (Ia S), correspondant au radical $R_1 = CH_3CO$- :

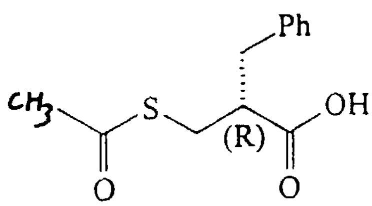
(IaR)

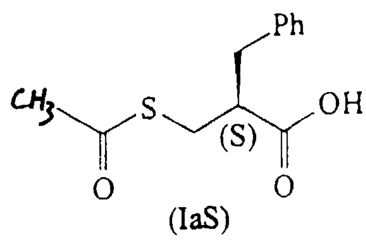
(IaS)

Appliqué à la synthèse de l'acide (R)-2-acétylthiométhyl-3-phényl propanoïque de formule (Ia R), le procédé conforme à l'invention comprend les étapes consistant à :

a) effectuer la réduction d'un ester d'acide malonique (V) tel que le benzylmalonate de diméthyle ($R_4=CH_3$), à l'aide d'un hydrure, tel que l'aluminohydrure de lithium, dans un solvant tel que le tétrahydrofuranne,
b) soumettre le diol (VI) formé à une monoacétylation énantiosélective à l'aide d'une enzyme, telle que la lipase PS (Amano) ou la lipase issue de Pseudomonas fluorescens (Fluka), dans un solvant tel que l'acétate de vinyle,
c) oxyder le monoacétate formé en présence d'un oxydant, tel que le réactif de Jones, dans un solvant tel que l'acétone,
d) effectuer une saponification par une solution aqueuse basique, telle qu'une solution aqueuse de soude ou une solution de lithine, suivie d'une acidification par une solution aqueuse acide, par exemple une solution aqueuse d'acide chlorhydrique, pour conduire à l'hydroxyacide de formule (XS) :

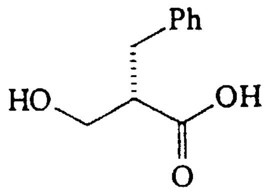

(XS)

g) et soumettre l'hydroxyacide (XS) à une réaction de substitution nucléophile de type Mitsunobu en présence, par exemple, d'un complexe d'azodicarboxylate d'alkyle/triphénylphosphine et d'acide thioacétique dans un solvant tel que le tétrahydrofuranne, pour former l'acide (Ia R).

Appliqué à la synthèse de l'acide (S)-2-acétylthiométhyl-3-phényl propanoïque de formule (Ia S), le procédé conforme à l'invention comprend les étapes consistant à :

b) effectuer la diacétylation du diol de formule (VI) soit par action de l'anhydride acétique en présence d'un catalyseur tel que, par exemple, le mélange 4-diméthylaminopyridine/triéthylamine ou l'acide sulfurique, soit à l'aide d'une enzyme telle que la Novozym 435 (Novo Nordisk) dans un solvant, par exemple, l'acétate de vinyle,

soumettre le diacétate formé à une mono-hydrolyse énantiosélective à l'aide d'une enzyme, telle la lipase PS (Amano) ou la lipase issue de Pseudomonas fluorescens (Fluka), dans un milieu tamponné, par exemple un tampon phosphate 0,1M pH7 et dans un solvant organique tel que l'acétone,

c) oxyder le monoacétate formé en présence d'un oxydant, tel que le réactif de Jones, dans un solvant tel que l'acétone,

d) effectuer une saponification par une solution aqueuse basique, telle qu'une solution aqueuse de soude ou une solution aqueuse de lithine, suivie d'une acidification par une solution aqueuse acide, par exemple une solution aqueuse d'acide chlorhydrique, pour conduire à l'hydroxyacide de formule (X R) :

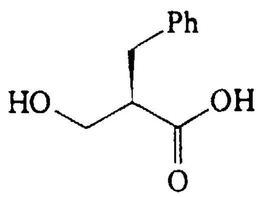

(XR)

e) et soumettre l'hydroxyacide (X R) à une réaction de substitution nucléophile de type Mitsunobu en présence, par exemple, d'un complexe d'azodicarboxylate d'alkyle/triphénylphosphine et d'acide thioacétique dans un solvant tel que le tétrahydrofuranne pour former l'acide (Ia S).

Les acides optiquement purs de formule (I S) de configuration (S) ou (I R) de configuration (R), obtenus par le procédé conforme à la présente invention, trouvent une utilisation particulièrement avantageuse dans la synthèse des N-(mercaptoacyl) amino-acides optiquement purs de formule (II).

Ils sont particulièrement appropriés à la synthèse des dérivés d'amino-acides de formule (III) et (IV).

La préparation des N-(mercaptoacyl) amino-acides à partir des acides de formule (I) est connue et est, par exemple, décrite dans le brevet européen n° EP 501 870.

Ainsi, les N-(mercaptoacyl) aminoacides peuvent être obtenus par la succession d'étapes données ci-dessous :

f) l'acide de formule (I), sous forme optiquement pure (I R) ou (I S), est transformé en chlorure d'acide (XII S) ou (XII R) à l'aide d'un agent de chloration, tel que le chlorure de thionyle ou le chlorure d'oxalyle :

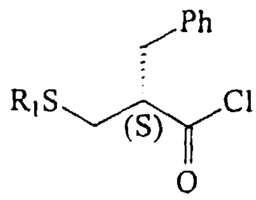

(XIIS)

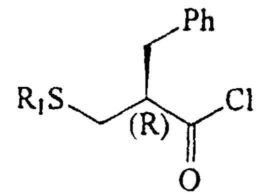

(XIIR)

$R_1$ ayant la même signification que dans la formule (I).

g) le chlorure d'acide de formule (XII) sous forme optiquement pure (XII S) ou (XII R) est ensuite couplé avec un amino ester de formule (XIII) :

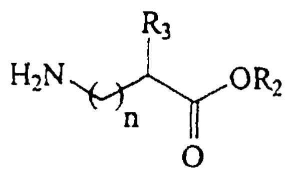

(XIII)

où $R_2$, $R_3$ et n ont les significations qui ont été données dans la formule (II), en présence d'une base telle que la triéthylamine, pour former les dérivés de formule (II) optiquement purs :

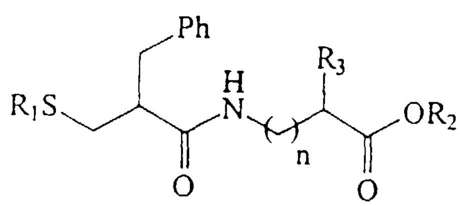

(II)

Il va être donné ci-après, à titre non limitatif, quelques exemples illustrant la mise en oeuvre du procédé conforme à l'invention.

**Exemple 1 : Acide (R)-2-acétylthiométhyl-3-phényl propanoïque (Ia R)**

<u>Stade a : 2-benzyl-1,3-propanediol (VI)</u>

Dans un erlenmeyer tricol d'un litre, on ajoute 15 g (395,2 mmoles) d'aluminohydrure de lithium à 200 ml de tétrahydrofuranne anhydre. On ajoute sous agitation une solution préparée à partir de 22,5 g (101,3 mmoles) de benzylmalonate de diméthyle et de 30 ml de tétrahydrofuranne anhydre de façon à avoir un léger reflux. On porte le milieu réactionnel trois heures au reflux. On refroidit avec un bain de glace, on dilue par 140 ml de tétrahydrofuranne et on hydrolyse par l'addition successive de 15 ml d'eau, 15 ml d'une solution aqueuse de soude à 15 % et 45 ml d'eau.

Après 0,5 heure d'agitation à température ambiante, le milieu réactionnel est blanc laiteux. Le précipité est filtré et lavé par 300 ml d'éther éthylique. La phase organique est séchée sur sulfate de magnésium, filtrée et concentrée. On obtient 16 g de solide que l'on triture dans 200 ml d'éther de pétrole glacé. Après filtration et séchage sous vide sur $P_2O_5$, on obtient 15 g (90,24 mmoles) de 2-benzyl-1,3-propanediol. Masse = 15 g

Rendement = 89 %

Fusion = 68°C (Köfler)

IR ($cm^{-1}$) : 3255

RMN $^1H(CDCl_3)$ : 7,4 à 7,0 (m, 5H) ; 3,9 à 3,5 (m, 4H) ; 3,1 (s large, 2H) ; 2,55 (d, 2H, J = 7 Hz) ; 2,1 à 1,9 (m, 1H).

RMN$^{13}C$ ($CDCl_3$) : 139,7 ; 128,9 ; 128,3 ; 126,0 ; 65,0 ; 43,7 ; 34,1.

Stade b : (R)-3-acétoxy-2-benzyl-propanol (VII R)

A une suspension de 4 g (24 mmoles) de 2-benzyl-1,3-propanediol (stade a) dans 40 ml d'acétate de vinyle, on ajoute à 25°C 10 mg de lipase PS (Amano) et on agite 48 heures à cette température. On filtre le milieu réactionnel et on concentre.

On obtient ainsi l'acétate de (R)-3-acétoxy-2-benzyl-propanol sous forme d'une huile.
Masse = 4,29 g
Rendement = 86 %
$[\alpha]_D^{20}$=+28,5° ($CHCl_3$, c=1,15)
IR ($cm^{-1}$) : 1735
RMN $^1H$ ($CDCl_3$) : 7,35 à 7,10 (m, 5H) ; 4,25 à 4,0 (m, 2H) ; 3,65 à 3,40 (m, 2H) ; 2,75 à 2,50 (m, 2H) ; 2,20 à 2,0 (m, 2H) ; 2,05 (s, 3H).
RMN $^{13}C$($CDCl_3$) : 190,7 ; 138,1 ; 128,9 ; 128,4 ; 126,2 ; 63,6 ; 61,9 ; 42,5 ; 34,2 ; 20,8.

Stade c : acide (S)-2-benzyl-3-acétoxypropanoïque (IX S)

A 4,0 g (19,2 mmoles) de (R)-3-acétoxy-2-benzyl-propanol (VII R) précédent en solution dans 80 ml d'acétone, on ajoute goutte à goutte à 0°C 22,8 ml de réactif de Jones. On agite ensuite 10 minutes à 0°C, puis on additionne à cette température 10 ml d'isopropanol. On maintient l'agitation une heure à 0°C.

Le milieu réactionnel est filtré. Le filtrat est dilué avec 40 ml d'eau, puis concentré sous vide afin d'éliminer l'acétone. Le résidu est basifié à froid (0, + 10°C) avec de l'hydrogénocarbonate de sodium jusqu'à pH = 9. La phase aqueuse basique est alors lavée avec de l'acétate d'éthyle (trois fois 15 ml).

La phase aqueuse est ensuite refroidie à l'aide d'un bain glace-eau et acidifiée jusqu'à pH = 1 avec une solution d'acide chlorhydrique concentré.

La phase aqueuse est extraite avec de l'acétate d'éthyle (trois fois 15 ml).

Les phases d'extraction sont réunies, lavées à l'eau (une fois 10 ml), séchées sur sulfate de magnésium, filtrées et concentrées sous vide.

On obtient ainsi l'acide (S)-2-benzyl-3-acétoxypropanoïque (IX S) sous forme d'huile.
Masse = 2,8 g
Rendement = 65 %
$[\alpha]_D^{20}$=+11,5° ($CHCl_3$, c=0,98)
IR ($cm^{-1}$) : 2 960, 1 740, 1 705
RMN $^1H$ ($CDCl_3$) : 10,4 (s large, 1H) ; 7,35 à 7,1 (m, 5H) ; 4,3 à 4,15 (m, 2H) ; 3,15 à 2,95 (m, 2H) ; 2,95 à 2,75 (m, 1H) ; 2,05 (s, 3H).
RMN $^{13}C$($CDCl_3$) : 178,5 ; 170,6 ; 137,4 ; 128,7 ; 128,5 ; 126,7 ; 63,4 ; 46,1 ; 34,3 ; 20,8.

Stade d : acide (S)-2-benzyl-3-hydroxypropanoïque (X S)

A une solution de 3,52 g (15,84 mmoles d'acide (S)-2-benzyl-3-acétoxypropanoïque (IX S) et de 35 ml d'un mélange de tétrahydrofuranne et d'eau (75/25), on ajoute à 0°C 2,64 g (62,9 mmoles) d'hydroxyde de lithium monohydraté. On agite encore une heure à 0°C. On acidifie avec 22 ml d'une solution aqueuse d'acide chlorhydrique 3M puis extrait avec de l'éther éthylique (une fois 50 ml, deux fois 15 ml).

Les phases éthérées sont réunies, séchées sur sulfate de magnésium, filtrées et concentrées sous vide. Le résidu solide obtenu est trituré à environ 5°C avec de l'éther de pétrole. On filtre, on essore et on sèche sous vide.
Masse = 2,45 g
Rendement = 86 %
Point de fusion = 63 - 65°C
$[\alpha]_D^{20}$=-14,3°(c=1,15 dans le chloroforme)
IR ($cm^{-1}$) : 1 705
RMN $^1H$ ($CDCl_3$) : 7,4 à 7,1 (m, 5H) ; 5,85 (s large, 2H) ; 3,85 à 3,6 (m, 2H) ; 3,15 à 2,95 (m, 1H) ; 2,95 à 2,75 (m, 2H).
RMN $^{13}C$ ($CDCl_3$) : 178,3 ; 138,2 ; 128,8 ; 128,5 ; 126,5 ; 61,9 ; 48,8 ; 33,9.
Pureté optique ≥95 %

Stade e : acide(R)-2-acétylthiométhyl-3-phénylpropanoïque (Ia R)

A une solution de 3,16 g (12,05 mmoles de triphénylphosphine dans 30 ml de tétrahydrofuranne, on ajoute goutte à goutte à 0°C 2,44 g (12,05 mmoles) d'azodicarboxylate de diisopropyle. Une fois l'addition terminée, on agite 30 minutes à cette température. On refroidit le milieu réactionnel à -10°C et ajoute goutte à goutte une solution de

1,45 g (8,05 mmoles) d'acide (S)-2-benzyl-3-hydroxypropionique (X S) et de 0,92 g (12,05 mmoles) d'acide thioacétique dans 10 ml de tétrahydrofuranne. Une fois l'addition terminée, on agite une heure à -10°C et encore deux heures à température ambiante.

On évapore le milieu réactionnel sous vide et on ajoute 25 ml d'une solution aqueuse saturée d'hydrogénocarbonate de sodium, puis 15 ml d'acétate d'éthyle. Après cinq minutes d'agitation vive, on laisse décanter et on recueille la phase aqueuse. La phase organique est ensuite de nouveau extraite par 20 ml d'une solution aqueuse saturée d'hydrogénocarbonate de sodium. Les phases aqueuses basiques sont réunies, lavées par 10 ml d'acétate d'éthyle et acidifiées à pH 1 avec 3 ml d'acide chlorhydrique concentré.

La phase aqueuse acide est ensuite extraite avec de l'acétate d'éthyle (2 x 15 ml).

Les phases organiques sont réunies, séchées sur sulfate de magnésium, filtrées et concentrées sous vide.

On obtient ainsi l'acide (R)-2-acétylthiométhyl-3-phényl propanoïque (Ia R) sous forme d'huile.
Masse = 1,57 g
Rendement = 82 %
$[\alpha]_D^{25}$=+33,7° ($CH_3OH$, c=0,98)
IR ($cm^{-1}$) : 1700
RMN $^1H$ ($CDCl_3$) : 9,5 (s large, 1 H) ; 7,40 à 7,10 (m, 5H) ; 3,20 à 2,80 (m, 5H) ; 2,30 (s, 3H).
RMN $^{13}C$($CDCl_3$) : 195,1 ; 179,3 ; 137,3 ; 128,8 ; 128,4 ; 126,6 ; 46,8 ; 37,3 ; 30,3 ; 29,4.
ee ≥95 %

* <u>Purification optique pour obtenir l'acide (R)-2-acétylthiométhyl-3-phényl propanoïque (Ia R)</u>

On place dans un ballon de 50 ml 1,54 g (6,5 mmoles) d'acide (R)-2-acétylthiométhyl-3-phényl propanoïque (Ia R) précédent et 15,4 ml d'éther éthylique. On agite jusqu'à dissolution, puis on ajoute 1,07 g (6,5 mmoles) de D(+)-éphédrine à température ambiante. On agite une heure à température ambiante. On filtre, on essore, on lave avec 5 ml d'éther éthylique glacé et on sèche sous vide. On obtient un sel blanc.
Masse = 2,41 g
Rendement = 92 %
$[\alpha]_D^{20}$=+48,4° ($CH_3OH$, c=0,95)

Le sel de (+)-éphédrine précédent est ensuite mis en suspension dans 10 ml d'éther éthylique. On ajoute 9 ml d'une solution aqueuse d'acide chlorhydrique 1N et on agite vivement jusqu'à dissolution du sel.

On laisse décanter et on recueille la phase éthérée. La phase organique est séchée sur sulfate de magnésium, filtrée et concentrée sous vide. On obtient ainsi l'acide (R)-2-acétylthiométhyl-3-phényl propanoïque (Ia R) optiquement pur, sous forme d'huile.
Masse = 1,37 g
Rendement = 89 %
$[\alpha]_D^{25}$=+35,7° ($CH_3OH$, c=1,3)

**Exemple 2 : Acide (S)-2-acétylthiométhyl-3-phényl propanoïque (Ia S)**

<u>Stade a : diacétate du 2-benzyl-1,3-propanediol (VIII)</u>

A une suspension de 1,89 g (11,37 mmoles) de 2-benzyl-1,3-propanediol (VI) dans 10 ml d'acétate de vinyle, on ajoute à 29°C 100 mg d'enzyme Novozym 435 et on agite 24 heures à cette température. On filtre le milieu réactionnel et on concentre.

On obtient ainsi le diacétate du 2-benzyl-1,3-propanediol (VIII) sous forme d'une huile.
Masse = 2,79 g
Rendement = 98 %
RMN $^1H$($CDCl_3$) : 7,35 à 7,10 (m, 5H) ; 4,15 à 3,90 (m, 4H) ; 2,75 à 2,60 (m, 2H) ; 2,40 à 2,25 (m, 1H) ; 2,05 (s, 6H).

<u>Stade b : (S)-3-acétoxy-2-benzyl-propanol (VII S)</u>

A 0,25 g (1 mmole) de diacétate du 2-benzyl-1,3-propanediol (VIII) précédent en solution dans un mélange de 9,9 ml d'acétone et de 23,1 ml de tampon phosphate pH = 7, on ajoute 130 mg de lipase issue de Pseudomonas fluorescens (Fluka), puis on chauffe le milieu à 30°C pendant 48 heures. La solution est ensuite extraite à l'éther éthylique (deux fois 20 ml). Les phases éthérées sont réunies, séchées sur sulfate de magnésium, filtrée et concentrées sous vide. Le résidu est purifié par chromatographie sur silice (éluant éther éthylique/éther de pétrole 7/3). On obtient 80 mg d'huile.
Rendement = 38 %

$[\alpha]_D^{20}$=-27,1° (c=1,04 dans le chloroforme)

Les caractéristiques spectrales sont identiques à celles du composé (VII R) (Exemple 1, stade b).

Stade c : acide (R)-2-benzyl-3-acétoxypropanoïque (IX R)

L'acétate (VII S) précédent est oxydé en acide selon le même mode opératoire que celui décrit à l'exemple 1, stade c.
Rendement = 68 %
$[\alpha]_D^{20}$=-11,3° (c=1,0 dans le chloroforme)

stade d : acide (R)-2-benzyl-3-hydroxypropanoïque (X R)

Le produit du stade c précédent est traité avec une solution aqueuse d'hydroxyde de lithium comme décrit à l'exemple 1, stade d.
Rendement = 91 %
$[\alpha]_D{}^0$=+14,1° (c=1,12 dans le chloroforme)
Pureté optique ≥ 95 %

Stade e : acide (S)-2-acétylthiométhyl-3-phénylpropanoïque (Ia S)

L'hydroxy-acide précédent est substitué par l'acide thioacétique selon une réaction de type Mitsunobu en utilisant le même mode opératoire que celui décrit à l'exemple 1, stade e pour conduire à l'acide (S)-2-acétylthiométhyl-3-phénylpropanoïque (Ia S). ee ≥95 %
Rendement après traitement avec de la (-)éphédrine = 70 %
$[\alpha]_D^{25}$=-34,8° (c=1,1 dans le méthanol)

**Exemple 3 : N-(R)-[2-acétylthiométhyl-1-oxo-3-phénylpropyl]-glycinate de benzyle (III)**

Stade f : chlorure de 2-acétylthiométhyl-3-phénylpropanoyle (XII S)

On place dans un ballon 2,0 g (8,40 mmoles) d'acide (R)-2-acétylthiométhyl-3-phényl-propanoïque (Ia R) (exemple 1, stade e). On refroidit par un bain glace/eau et ajoute goutte à goutte 1,2 g (10,08 mmoles) de chlorure de thionyle. On agite pendant une nuit à température ambiante.

On évapore l'excès de chlorure de thionyle à l'évaporateur rotatif. On obtient 2,15 g d'huile jaunâtre.
Rendement quantitatif
$[\alpha]_D^{25}$=+29,9° (c=1,30 dans le chloroforme)
IR ($cm^{-1}$): 1790 à 1780, 1695 à 1685
RMN $^1H$ ($CDCl_3$) : 7,4 à 7,1 (m, 5H) ; 3,5 à 2,8 (m, 5H) ; 2,25 (s, 3H).

Stade g : N-(R)-[2-acétylthiométhyl-1-oxo-3-phénylpropyl]-glycinate de benzyle (III)

On place dans un tricol muni d'une garde à chlorure de calcium 2,83 g (8,40 mmoles) de glycinate de benzyle sous forme de sel de paratoluène sulfonate dans 6 ml de dichlorométhane. On refroidit par un bain de glace/eau à 5°C.

On ajoute goutte à goutte à une température comprise entre 5 et 15°C une solution de 1,70 g (16,83 mmoles) de triéthylamine dans 5 ml de dichlorométhane. On agite 10 minutes, puis on ajoute une solution de 2,15 g (8,40 mmoles) du chlorure d'acide précédent dans 5 ml de dichlorométhane sans dépasser 15°C dans le milieu.

On laisse la température revenir à l'ambiante, puis on agite encore trois heures.

Le milieu réactionnel est lavé à l'eau (une fois 5 ml), par une solution aqueuse d'acide chlorhydrique normale (une fois 5 ml), par une solution aqueuse saturée d'hydrogénocarbonate de sodium (une fois 5 ml) et par une solution aqueuse saturée de chlorure de sodium (une fois 5 ml).

La phase organique est séchée sur sulfate de magnésium, filtrée et concentrée sous vide. On obtient 3,2 g de résidu solide que l'on dissout dans l'isopropanol à chaud (12 ml).

On filtre à chaud. Le filtrat est refroidi par un bain glace/eau. Le solide est filtré, lavé à l'éther diisopropylique, essoré et séché sous vide sur pentaoxyde de phosphore.
Masse obtenue = 2,1 g
Rendement = 65 %
Fusion : 69°C
$[\alpha]_D^{20}$=+24,5° (c=1,0 dans le méthanol)

IR (nujol) ($cm^{-1}$) : 3 280, 1 755, 1 695, 1 640
RMN $^1H$ ($CDCl_3$) : 7,40 à 7,10 (m, 10H) ; 6,15 (t large, 1H); 5,25 (s, 2H) ; 4,10 à 3,5 (AB dédoublé, 2H) ; 3,10 à 2,55 (m, 5H) ; 2,30 (s, 3H).
RMN$^{13}C$ ($CDCl_3$) : 195,8 ; 172,9 ; 169,2 ; 138,4 ; 135,0 ; 128,7 ; 128,4 ; 128,2 ; 126,5 ; 66,9 ; 49,1 ; 41,2 ; 38,2 ; 31,0 ; 30,4.

| Analyse élémentaire $C_{21}H_{23}O_4NS$ | | | |
|---|---|---|---|
| | C | N | H |
| % calculé | 65,45 | 3,63 | 5,97 |
| % trouvé | 65,34 | 3,95 | 6,10 |

**Exemple 4 : N-(S)-[2-acétylthiométhyl-1-oxo-3-phénylpropyl]-glycinate de benzyle (IV)**

Stade f : chlorure de 2-acétylthiométhyl-3-phénylpropanoyle (XII R)

L'acide (S)-2-acétylthiométhyl-3-phénylpropanoïque (Ia S) (Exemple 2, stade e) est traité avec du chlorure de thionyle selon le même mode opératoire que celui décrit à l'exemple 3, stade f.
Rendement quantitatif
$[\alpha]_D^{25}$=-30,1° (c=1,37 dans le chloroforme)

Stade g : N-(S)-[2-acétylthiométhyl-1-oxo-3-phénylpropyl]-glycinate de benzyle (IV)

Le chlorure d'acide précédent est couplé avec le glycinate de benzyle selon le même mode opératoire que celui décrit à l'exemple 3, stade g.
Rendement = 66 %
$[\alpha]_D^{20}$=-24,9° (c=1,0 dans le méthanol)

| Analyse élémentaire : $C_{21}H_{23}O_4NS$ | | | |
|---|---|---|---|
| | C | N | H |
| % calculé | 65,45 | 3,63 | 5,97 |
| % trouvé | 65,02 | 3,88 | 5,87 |

**Revendications**

1. Procédé de synthèse asymétrique des dérivés S-acylés de l'acide 2-mercaptométhyl 3-phényl propanoïque de formule (I) :

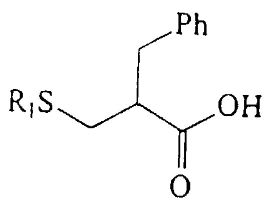 (I)

dans laquelle
$R_1$ représente un radical acyle aliphatique linéaire ou ramifié ou un radical acyle aromatique, caractérisé en ce qu'il comprend les étapes consistant à :

a) préparer le diol de formule (VI) :

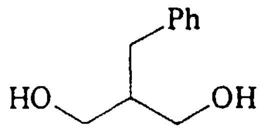 (VI)

par réduction d'un ester malonique de formule (V) :

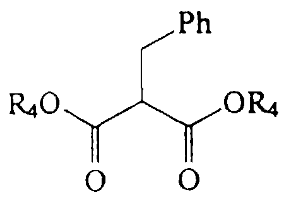 (V)

dans laquelle $R_4$ représente une chaîne alkyle contenant de 1 à 4 atomes de carbone, en présence d'un hydrure ;

b) préparer le monoacétate (VII) respectivement de configuration (R) et de configuration (S), de formules (VII R) ou (VII S) :

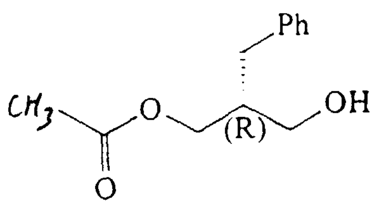

(VIIR)

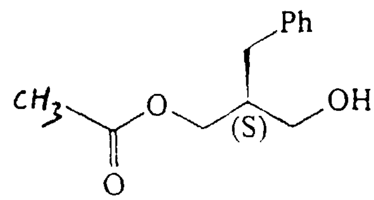

(VIIS)

c) soumettre les monoacétates de formule (VII R) ou (VII S) à une oxydation pour former les acides de formule (IX S) ou (IX R) :

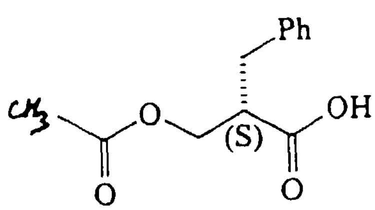

(IXS)

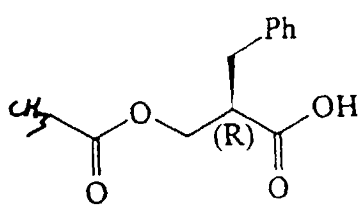

(IXR)

d) saponifier les composés de formule (IX S) ou (IX R), en présence d'une solution aqueuse basique pour former les hydroxyacides de formule (X S) ou (X R) :

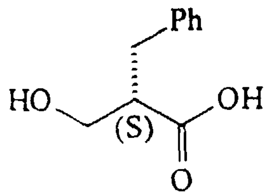

(XS)

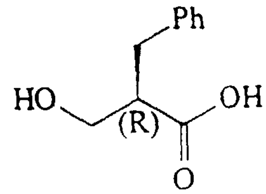

(XR)

e) thioacyler les hydroxyacides de formule (X S) ou (X R) avec un mercaptoacide de formule (XI) :

R1 S H (XI)

dans laquelle $R_1$ représente un radical acyle aliphatique linéaire ou ramifié ou un radical acyle aromatique, selon une réaction de type Mitsunobu en présence d'un complexe d'azodicarboxylate d'alkyle/triphénylphosphine pour conduire respectivement aux acides de formule (I R) ou (I S) désirés:

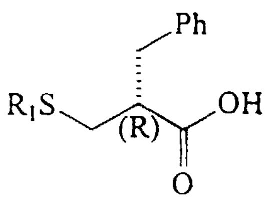

(IR)

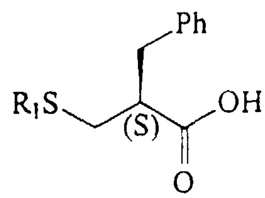

(IS)

**2.** Procédé selon la revendication 1, caractérisé en ce que le monoacétate (VII R) de configuration (R) est obtenu en faisant réagir le diol de formule (VI) avec de l'acétate de vinyle, en présence d'une enzyme.

**3.** Procédé selon la revendication 1, caractérisé en ce que le monoacétate (VII S) de configuration (S) est obtenu par préparation du diacétate de formule (VIII) :

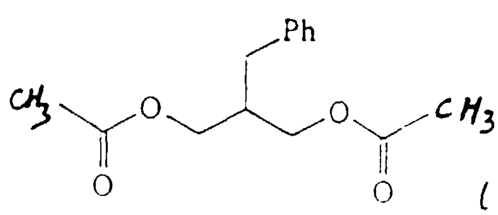

(VIII)

à partir du diol de formule (VI) puis par mono-hydrolyse énantiosélective du diacétate de formule (VIII) en présence d'une enzyme, pour former le monoacétate dérivé.

**4.** Procédé selon la revendication 3, caractérisé en ce que le diacétate de formule (VIII) est obtenu en faisant réagir le diol de formule (VI) avec de l'anhydride acétique en présence d'un catalyseur.

**5.** Procédé selon la revendication 3, caractérisé en ce que le diacétate de formule (VIII) est obtenu en faisant réagir

le diol de formule (VI) avec de l'acétate de vinyle en présence d'une enzyme.

**6.** Procédé selon l'une quelconque des revendications 1 à 5, caractérisé en ce que l'hydrure utilisé à l'étape a) est choisi parmi l'aluminohydrure de lithium et le borohydrure de sodium.

**7.** Procédé selon la revendication 2, caractérisé en ce que l'enzyme utilisée à l'étape b) pour la préparation du monoacétate (VII R) est choisie parmi la lipase PS (Amano) et la lipase issue de Pseudomonas fluorescens (Fluka).

**8.** Procédé selon l'une quelconque des revendications 3 et 4, caractérisé en ce que le catalyseur utilisé à l'étape b) pour la diacétylation du diol (VI) est choisi parmi le mélange 4-diméthylaminopyridine/triéthylamine et l'acide sulfurique.

**9.** Procédé selon l'une quelconque des revendications 3 et 5, caractérisé en ce que l'enzyme utilisée à l'étape b) pour la réaction de diacétylation du diol (VI) est la Novozym 435 (Novo Nordisk).

**10.** Procédé selon la revendication 3, caractérisé en ce que l'enzyme utilisée à l'étape b) pour la monohydrolyse du diacétate (VIII) est choisie parmi la lipase issue de Pseudomonas fluorescens (Fluka) et la lipase Ps (Amano).

**11.** Procédé selon l'une des revendications précédentes, caractérisé en ce que l'oxydant utilisé à l'étape c) est choisi parmi le réactif de Jones, le permanganate de potassium et l'acide nitrique.

**12.** Procédé selon l'une quelconque des revendications précédentes, caractérisé en ce que la solution aqueuse basique utilisée à l'étape d) est choisie parmi une solution aqueuse d'hydroxyde de lithium et une solution aqueuse d'hydroxyde de sodium.

**13.** Procédé selon l'une quelconque des revendications précédentes, caractérisé en ce que l'azodicarboxylate d'alkyle utilisé à l'étape e) pour la réaction de Mitsunobu, est choisi parmi l'azodicarboxylate de diisopropyle et l'azodicarboxylate de diéthyle.

**14.** Procédé selon l'une quelconque des revendications précédentes, caractérisé en ce que le mercaptoacide de formule (XI) utilisé à l'étape e) pour la réaction de Mitsunobu, est choisi parmi l'acide thioacétique et l'acide thiobenzoïque.

**15.** Procédé selon l'une quelconque des revendications précédentes, caractérisé en ce qu'il comprend les étapes consistant à, lorsqu'il est appliqué à la préparation de l'acide (R)-2-acétylthiométhyl-3-phényl propanoïque ($R_1$=acétyl) de formule (Ia R) :

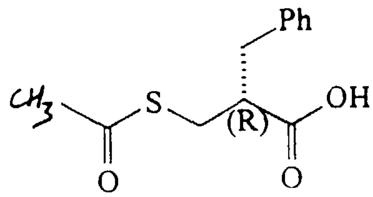
(IaR)

a) effectuer une réduction d'un ester d'acide malonique tel que le benzylmalonate de diméthyle ($R_4$ = $CH_3$), à l'aide d'un hydrure tel que l'aluminohydrure de lithium, dans un solvant tel que le tétrahydrofuranne, pour obtenir le diol (VI) correspondant ;
b) effectuer une monoacétylation du diol de formule (VI) ainsi obtenu dans l'acétate de vinyle, en présence de lipase PS (Amano),
c) oxyder la fonction alcool du monoacétate de formule (VII R) ainsi obtenu, dans l'acétone, à l'aide du réactif de Jones ($CrO_3$ - $H_2SO_4$),
d) saponifier la fonction acétate par une solution aqueuse alcaline, telle qu'une solution aqueuse de lithine, puis libérer par acidification l'hydroxyacide de formule (X S) :

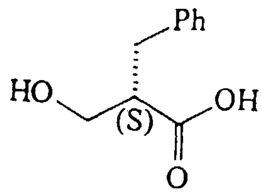

(XS)

e) substituer la fonction alcool de l'hydroxyacide de formule (X S) selon une réaction de type Mitsunobu en présence du complexe triphénylphosphine/azodicarboxylate de diisopropyle et d'acide thioacétique pour donner l'acide de configuration (R) de formule (Ia R).

**16.** Procédé selon l'une quelconque des revendications 1 à 14, caractérisé en ce qu'il comprend les étapes consistant à, lorsqu'il est appliqué à la préparation de l'acide (S)-2-acétylthiométhyl-3-phényl propanoïque de formule (Ia S) :

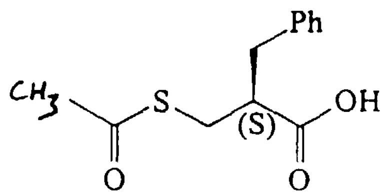

(IaS)

a) effectuer une réduction d'un malonate (V) tel que le benzylmalonate de diméthyle ($R_4 = CH_3$), à l'aide d'un hydrure, tel que l'aluminohydrure de lithium,
b) effectuer une diacétylation du diol de formule (VI) dans l'acétate de vinyle, en présence d'enzyme Novozym 435 (Novo Nordisk), puis effectuer une monohydrolyse du diacétate de formule (VIII) ainsi formé dans du tampon phosphate PH 7, en présence de lipase issue de Pseudomonas fluorescens (Fluka),
c) oxyder la fonction alcool du monoacétate de formule (VII S) dans l'acétone, à l'aide du réactif de Jones ($CrO_3$ - $H_2SO_4$),
d) saponifier la fonction acétate par une solution aqueuse alcaline, telle qu'une solution aqueuse de lithine, puis libérer par acidification l'hydroxyacide de formule (X R) :

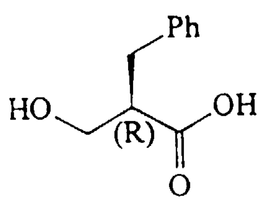

(XR)

e) substituer la fonction alcool de l'hydroxyacide de formule (X R) selon une réaction de type Mitsunobu en présence d'un complexe triphénylphosphine/azodicarboxylate de diisopropyle et d'acide thioacétique pour donner l'acide de configuration (S) de formule (la S).

**17.** Procédé de préparation de dérivés N-(mercaptoacyl)amino-acides optiquement actifs de formule (II) :

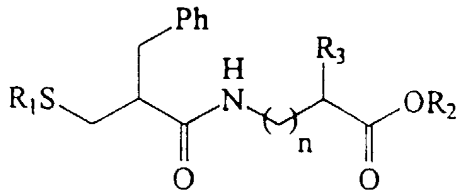

(II)

dans laquelle

$R_1$ représente un atome d'hydrogène, un radical acyle aliphatique linéaire ou ramifié ou un radical acyle aromatique,
$R_2$ représente un atome d'hydrogène, un radical alkyle inférieur, un radical phényle ou un groupe phénylalkylène inférieur,
$R_3$ représente un atome d'hydrogène ; un groupe alkyle inférieur ; un groupe hydroxylalkylène inférieur ; un groupe phényle ; un groupe phénylalkylène inférieur ; un groupe hydroxyphénylalkylène inférieur ; un groupe aminoalkylène inférieur ; un groupe guanidinoalkylène inférieur ; un groupe mercaptoalkylène inféreur ; un groupe alkyle inférieur thioalkylène inférieur ; un groupe imidazolyalkylène inférieur ; un groupe indolylalkylène inférieur ; un groupe carbamylalkylène inférieur ; un groupe carboxyalkylène inférieur ;
n varie de 0 à 10 ;

caractérisé en ce qu'il comprend l'utilisation des dérivés S-acylés de l'acide 2-mercaptométhyl 3-phényl propanoique de formule (I) sous forme optiquement pure, après leur obtention à l'aide du procédé des revendications 1 à 16.

**18.** Procédé selon la revendication 17, caractérisé en ce qu'il comprend l'utilisation du composé de formule (Ia R) ou (Ia S).

**19.** Procédé selon la revendication 17, caractérisé en ce que l'on utilise le composé (Ia R) pour la synthèse du composé de formule (III):

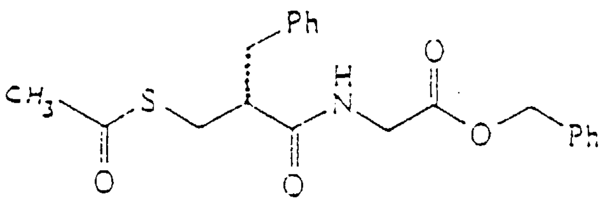

(III)

**20.** Procédé selon la revendication 17, caractérisé en ce que l'on utilise le composé (Ia S) pour la synthèse du composé de formule (IV):

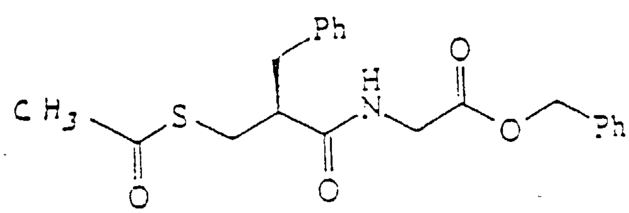

(IV)

**21.** Procédé selon l'une quelconque des revendications 17 à 20, caractérisé en ce qu'il comprend les étapes consistant à :

f) former le chlorure (XII S) ou (XII R)

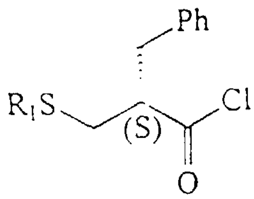

(XIIS)

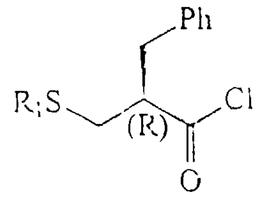

(XIIR)

de l'acide (I R) ou (I S) à l'aide d'un agent de chloration et

g) faire réagir le chlorure d'acide (XII S) ou (XII R) avec un aminoester de formule (XIII)

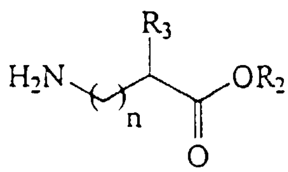

(XIII)

en présence d'une base.

**22.** Utilisation des dérivés S-acylés de l'acide optiquement pur 2-mercaptométhyl-3-phényl propanoïque de formule (I) obtenu par le procédé selon l'une quelconque des revendications 1 à 16 pour la synthèse de dérivés N-(mercaptoacyl) amino-acides optiquement purs de formule (II) :

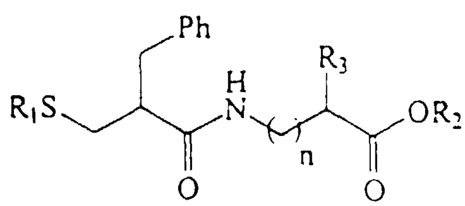

(II)

dans laquelle

$R_1$ représente un atome d'hydrogène, un radical acyle aliphatique linéaire ou ramifié ou un radical acyle aromatique,

$R_2$ représente un atome d'hydrogène, un radical alkyle inférieur, un radical phényle ou un groupe phénylalkylène inférieur,

$R_3$ représente un atome d'hydrogène ; un groupe alkyle inférieur ; un groupe hydroxylalkylène inférieur ; un groupe phényle ; un groupe phénylalkylène inférieur ; un groupe hydroxyphénylalkylène inférieur ; un groupe aminoalkylène inférieur ; un groupe guanidinoalkylène inférieur ; un groupe mercaptoalkylène inféreur ; un groupe alkyle inférieur thioalkylène inférieur ; un groupe imidazolyalkylène inférieur ; un groupe indolylalkylène inférieur ; un groupe carbamylalkylène inférieur ; un groupe carboxyalkylène inférieur ;

n varie de 0 à 10.

**23.** Utilisation selon la revendication 22 de l'acide (R)-2-acétylthiométhyl-3-phényl propanoïque de formule (Ia R) pour la synthèse des dérivés de formule (II).

**24.** Utilisation selon la revendication 23 de l'acide (R)-2-acétylthiométhyl-3-phényl propanoïque de formule (Ia R) pour la synthèse du N-(R)-[2-acétylthiométhyl-1-oxo-3-phénylpropyl]-glycinate de benzyle de formule (III).

**25.** Utilisation selon la revendication 22 de l'acide (S)-2-acétylthiométhyl-3-phényl propanoïque de formule (Ia S) pour la synthèse des dérivés de formule (II).

**26.** Utilisation selon la revendication 25 de l'acide (S)-2-acétylthiométhyl-3-phényl propanoïque de formule (Ia S) pour la synthèse du N-(S)-[2-acétylthiométhyl-1-oxo-3-phénylpropyl]-glycinate de benzyle de formule (IV).

**Patentansprüche**

**1.** Verfahren zur asymmetrischen Synthese von S-acylierten Derivaten von 2-Mercaptomethyl-3-phenyl-propansäure der Formel (I):

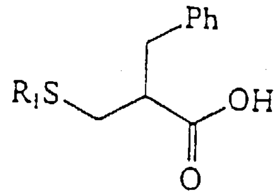 (I)

wobei
$R_1$ einen linearen oder verzweigten aliphatischen Acylrest oder einen aromatischen Acylrest darstellt, dadurch charakterisiert, daß es die Schritte umfaßt, welche bestehen aus:

a) Herstellung des Diols der Formel (VI):

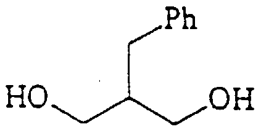 (VI)

durch Reduktion eines Malonesters der Formel (V):

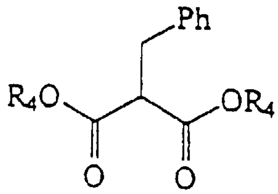 (V)

wobei $R_4$ eine Alkylkette darstellt, die 1 bis 4 Kohlenstoffatome umfaßt, in Gegenwart eines Hydrids;
b) Herstellung des Monoacetats (VII), jeweils der Konfiguration (R) und der Konfiguration (S), der Formel (VII R) oder (VII S):

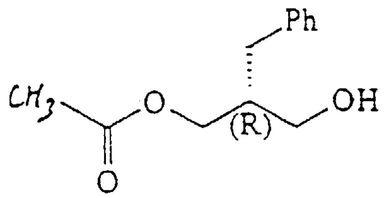

(VIIR)

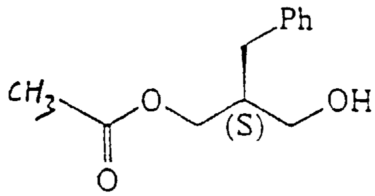

(VIIS)

c) Durchführung einer Oxidation mit den Monoacetaten der Formel (VII R) oder (VII S) zur Bildung von Säuren der Formel (IX S) oder (IX R):

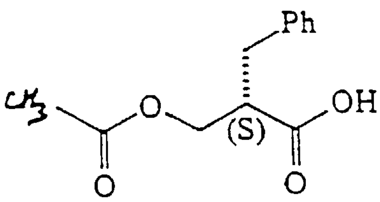

(IXS)

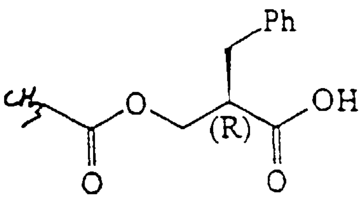

(IXR)

d) Verseifen der Verbindungen der Formel (IX S) oder (IX R) in Gegenwart einer wäßrigen basischen Lösung zur Bildung der Hydroxysäuren der Formel (X S) oder (X R):

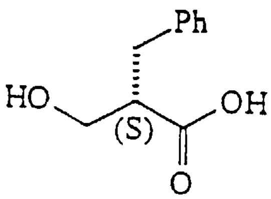

(XS)

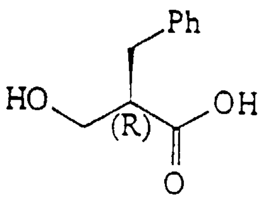

(XR)

e) Thioacylierung der Hydroxysäuren der Formel (X S) oder (X R) mit einer Mercaptosäure der Formel (XI):

R1 S H (XI)

wobei $R_1$ einen linearen oder verzweigten aliphatischen Acylrest oder einen aromatischen Acylrest darstellt, nach einer Reaktion vom Mitsunobu-Typ in Gegenwart eines Komplexes aus Alkylazodicarboxylat/Triphenylphosphin zur jeweiligen Herstellung der gewünschten Säuren der Formeln (I R) oder (I S):

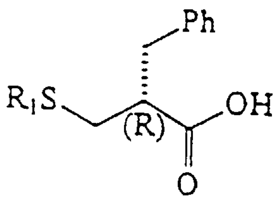

(IR)

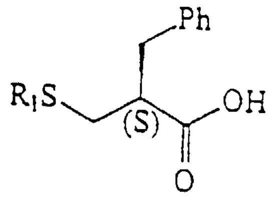

(IS)

2. Verfahren nach Anspruch 1, dadurch charakterisiert, daß das Monoacetat (VII R) der Konfiguration (R) erhalten wird, indem das Diol der Formel (VI) mit Vinylacetat in Gegenwart eines Enzyms umgesetzt wird.

3. Verfahren nach Anspruch 1, dadurch charakterisiert, daß das Monoacetat (VII S) der Konfiguration (S) erhalten wird durch Herstellung des Diacetats der Formel (VIII):

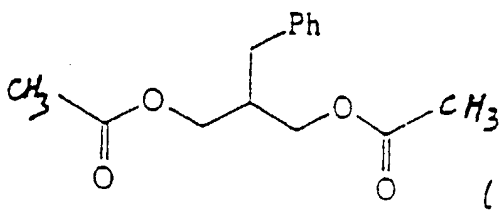

(VIII)

ausgehend vom Diol der Formel (VI) und anschließend durch enantioselektive Mono-Hydrolyse des Diacetats der Formel (VIII) in Gegenwart eines Enzyms zur Bildung des abgeleiteten Monoacetats.

4. Verfahren nach Anspruch 3, dadurch charakterisiert, daß das Diacetat der Formel (VIII) erhalten wird, indem das Diol der Formel (VI) mit Acetanhydrid in Gegenwart eines Katalysators umgesetzt wird.

5. Verfahren nach Anspruch 3, dadurch charakterisiert, daß das Diacetat der Formel (VIII) erhalten wird, indem das Diol der Formel (VI) mit Vinylacetat in Gegenwart eines Enzyms umgesetzt wird.

6. Verfahren nach einem der Ansprüche 1 bis 5, dadurch charakterisiert, daß das in Schritt a) verwendete Hydrid ausgewählt wird aus Lithiumaluminiumhydrid und Natriumborhydrid.

7. Verfahren nach Anspruch 2, dadurch charakterisiert, daß das in Schritt b) zur Herstellung des Monoacetats (VII R) verwendete Enzym ausgewählt wird aus Lipase PS (Amano) und aus Pseudomonas fluoreszens stammender Lipase (Fluka).

8. Verfahren nach einem der Ansprüche 3 und 4, dadurch charakterisiert, daß der in Schritt b) für die Diacetylierung des Diols (VI) verwendete Katalysator ausgewählt wird aus der Mischung 4-Dimethylaminopyridin/Triethylamin und Schwefelsäure.

9. Verfahren nach einem der Ansprüche 3 und 5, dadurch charakterisiert, daß das in Schritt b) für die Diacetylierungsreaktion des Diols (VI) verwendete Enzym Novozym 435 (Novo Nordisk) ist.

10. Verfahren nach Anspruch 3, dadurch charakterisiert, daß das in Schritt b) für die Monohydrolyse des Diacetats (VIII) verwendete Enzym ausgewählt wird aus der Gruppe, bestehend aus aus Pseudomonas fluoreszens stammender Lipase (Fluka) und Lipase PS (Amano).

11. Verfahren nach einem der vorstehenden Ansprüche, dadurch charakterisiert, daß das in Schritt c) verwendete Oxidationsmittel ausgewählt wird aus dem Jones-Reagens, Kaliumpermanganat und Salpetersäure.

**12.** Verfahren nach einem der vorstehenden Ansprüche, dadurch charakterisiert, daß die in Schritt d) verwendete wäßrige basische Lösung ausgewählt wird aus einer wäßrigen Lösung von Lithiumhydroxid und einer wäßrigen Lösung von Natriumhydroxid.

**13.** Verfahren nach einem der vorstehenden Ansprüche, dadurch charakterisiert, daß das in Schritt e) für die Mitsunobu-Reaktion verwendete Alkylazodicarboxylat ausgewählt wird aus Diisopropylazodicarboxylat und Diethylazodicarboxylat.

**14.** Verfahren nach einem der vorstehenden Ansprüche, dadurch charakterisiert, daß die in Schritt e) für die Mitsunobu-Reaktion verwendete Mercaptosäure ausgewählt wird aus Thioessigsäure und Thiobenzoesäure.

**15.** Verfahren nach einem der vorstehenden Ansprüche, dadurch charakterisiert, daß es, wenn es für die Herstellung von (R) 2-Acetylthiomethyl-3-phenyl-propansäure ($R_1$ = Acetyl) der Formel (Ia R) verwendet wird, die Schritte umfaßt, welche bestehen aus:

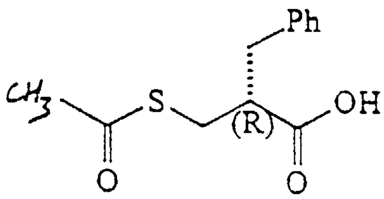

(IaR)

a) Durchführung einer Reduktion eines Malonsäureesters wie Dimethylbenzylmalonat ($R_4 = CH_3$) mit Hilfe eines Hydrids wie Lithiumaluminiumhydrid in einem Lösungsmittel wie Tetrahydrofuran, zur Herstellung des entsprechenden Diols (VI);
b) Durchführung einer Monoacetylierung des so erhaltenen Diols der Formel (VI) in Vinylacetat in Gegenwart von Lipase PS (Amano),
c) Oxidieren der Alkoholfunktion des so erhaltenen Monoacetats der Formel (VII R) in Aceton mit Hilfe des Jones-Reagens ($CrO_3$-$H_2SO_4$),
d) Verseifen der Acetatfunktion durch eine alkalische wäßrige Lösung wie einer wäßrigen Lithiumhydroxid-Lösung, anschließend Freisetzung der Hydroxysäure der Formel (X S) durch Ansäuern:

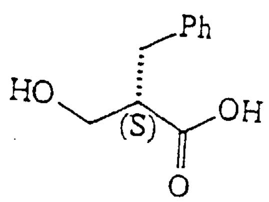

(XS)

e) Substituieren der Alkoholfunktion der Hydroxysäure der Formel (X S) mit einer Reaktion vom Typ Mitsunobu in Gegenwart eines Komplexes Triphenylphosphin/Diisopropylazodicarboxylat und von Thioessigsäure zur Herstellung der Säure der Konfiguration (R) der Formel (Ia R).

**16.** Verfahren nach einem der Ansprüche 1 bis 14, dadurch charakterisiert, daß es, wenn es zur Herstellung der Säure (S)-2-Acetylthiomethyl-3-phenylpropansäure der Formel (Ia S) verwendet wird:

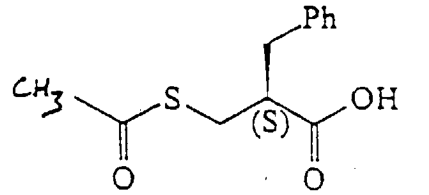 (IaS)

die Schritte umfaßt, die bestehen aus:

a) Durchführung einer Reduktion eines Malonats (V) wie Dimethylbenzylmalonat (R4 = $CH_3$) mit Hilfe eines Hydrids wie Lithiumaluminiumhydrid,
b) Durchführung einer Diacetylierung des Diols der Formel (VI) in Vinylacetat in Gegenwart des Enzyms Novozym 435 (Novo Nordisk), anschließend Durchführung einer Monohydrolyse des so gebildeten Diacetats der Formel (VIII) in Phosphatpuffer pH 7 in Gegenwart von aus Pseudomonas fluoreszens stammender Lipase (Fluka),
c) Oxidieren der Alkoholfunktion des Monoacetats der Formel (VII S) in Aceton mit Hilfe des Jones-Reagens ($CrO_3$-$H_2SO_4$),
d) Verseifen der Acetatfunktion durch eine alkalische wäßrige Lösung wie eine wäßrige Lithiumhydroxid-Lösung, anschließend Freisetzung der Hydroxysäure der Formel (X R) durch Ansäuern:

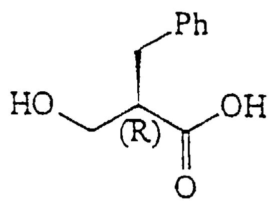 (XR)

e) Substituieren der Alkoholfunktion der Hydroxysäure der Formel (X R) mit einer Reaktion vom Typ Mitsunobu in Gegenwart eines Komplexes aus Triphenylphosphin/Diisopropylazodicarboxolat und von Thioessigsäure zur Herstellung der Säure der Konfiguration (S) der Formel (Ia S).

**17.** Verfahren zur Herstellung von optische aktiven N-(Mercaptoacyl)amino-Säure-Derivaten der Formel (II):

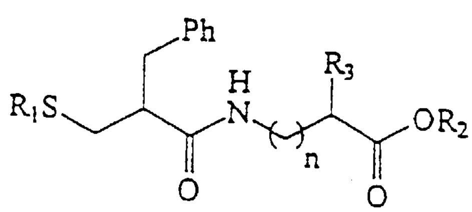 (II)

wobei

$R_1$ ein Wasserstoffatom, einen linearen oder verzweigten aliphatischen Acylrest oder einen aromatischen Acylrest darstellt,
$R_2$ ein Wasserstoffatom, einen niedrigen Alkylrest, einen Phenylrest oder eine niedrige Phenylalkylengruppe darstellt,
$R_3$ ein Wasserstoffatom; eine niedrige Alkylgruppe; eine niedrige Hydroxyalkylengruppe; eine Phenylgruppe;

eine niedrige Phenylalkylengruppe; eine niedrige Hydroxyphenylalkylengruppe; eine niedrige Aminoalkylengruppe; eine niedrige Guanidinoalkylengruppe; eine niedrige Mercaptoalkylengruppe; eine niedrige Alkyl-niedrige Thioalkylengruppe; eine niedrige Imidazolylalkylengruppe; eine niedrige Indolylalkylengruppe; eine niedrige Carbamoylalkylengruppe; eine niedrige Carboxyalkylengruppe darstellt;
n von 0 bis 10 variiert;

dadurch charakterisiert, daß es Verwendung der S-Acylierten Derivate von 2-Mercaptomethyl-3-phenylpropansäure der Formel (I) in optisch reiner Form nach deren Herstellung mit Hilfe des Verfahrens der Ansprüche 1 bis 16 umfaßt.

**18.** Verfahren nach Anspruch 17, dadurch charakterisiert, daß es Verwendung der Verbindung der Formel (Ia R) oder (Ia S) umfaßt.

**19.** Verfahren nach Anspruch 17, dadurch charakterisiert, daß man die Verbindung (Ia R) für die Synthese der Verbindung der Formel (III) verwendet:

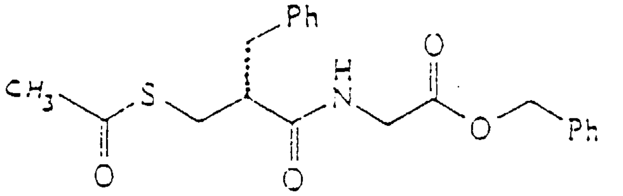
(III)

**20.** Verfahren nach Anspruch 17, dadurch charakterisiert, daß man die Verbindung (Ia S) für die Synthese der Verbindung der Formel (IV) verwendet:

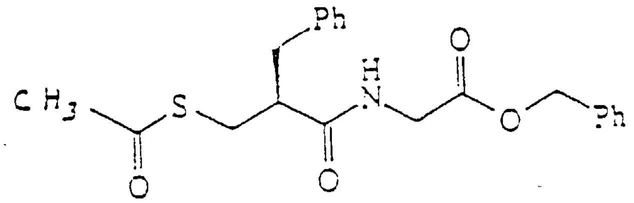
(IV)

**21.** Verfahren nach einem der Ansprüche 17 bis 20, dadurch charakterisiert, daß es die Schritte umfaßt, welche bestehen aus:

f) Bildung des Chlorids (XII S) oder (XII R)

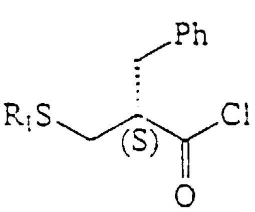
(XIIS)

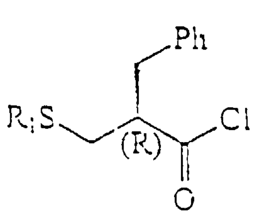
(XIIR)

der Säure (I R) oder (I S) mit Hilfe eines Chlorierungsmittels und
g) Umsetzung des Säurechlorids (XII S) oder (XII R) mit einem Aminoester der Formel (XIII)

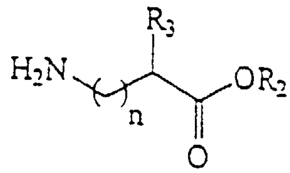

(XIII)

in Gegenwart einer Base.

**22.** Verwendung von S-acylierten Derivaten von optisch reiner 2-Mercaptomethyl-3-phenylpropansäure der Formel (I), erhalten durch das Verfahren nach einem der Ansprüche 1 bis 16, für die Synthese von optisch reiner N-(Mercaptoacyl)aminosäure-Derivaten der Formel (II):

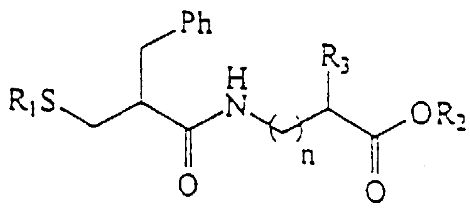

(II)

wobei

$R_1$ ein Wasserstoffatom, einen linearen oder verzweigten aliphatischen Acylrest oder einen aromatischen Acylrest darstellt,
$R_2$ ein Wasserstoffatom, einen niedrigen Alkylrest, einen Phenylrest oder eine niedrige Phenylalkylengruppe darstellt,
$R_3$ ein Wasserstoffatom; eine niedrige Alkylgruppe; eine niedrige Hydroxyalkylengruppe; eine Phenylgruppe; eine niedrige Phenylalkylengruppe; eine niedrige Hydroxyphenylalkylengruppe; eine niedrige Aminoalkylengruppe; eine niedrige Guanidinoalkylengruppe; eine niedrige Mercaptoalkylengruppe; eine niedrige Alkyl-niedrige Thioalkylengruppe; eine niedrige Imidazolylalkylengruppe; eine niedrige Indolylalkylengruppe; eine niedrige Carbamoylalkylengruppe; eine niedrige Carboxyalkylengruppe darstellt;
n von 0 bis 10 variiert.

**23.** Verwendung von (R)-2-Acetylthiomethyl-3-phenylpropansäure der Formel (Ia R) für die Synthese von Derivaten der Formel (II).

**24.** Verwendung von (R)-2-Acetylthiomethyl-3-phenylpropansäure der Formel (Ia R) nach Anspruch 23 für die Synthese von N-(R)-[2-Acetylthiomethyl-1-oxo-3-phenylpropyl]benzylglycinat der Formel (III).

**25.** Verwendung von (S)-2-Acetlythiomethyl-3-phenylpropansäure der Formel (Ia S) nach Anspruch 23 für die Synthese von Derivaten der Formel (II).

**26.** Verwendung von (S)-2-Acetylthiomethyl-3-phenylpropansäure der Formel (Ia S) nach Anspruch 25 für die Synthese von N-(S)-[2-Acetylthiomethyl-1-oxo-3-phenylpropyl]benzylglycinat der Formel (IV).

## Claims

**1.** Process for the asymmetric synthesis of S-acyl derivatives of 2-mercaptomethyl-3-phenylpropanoic acid of the formula (I):

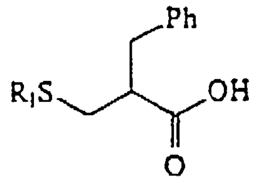 (I)

in which
$R_1$ represents a linear or branched aliphatic acyl radical or an aromatic acyl radical,
characterized in that it comprises the steps of:

a) preparing the diol of the formula (VI):

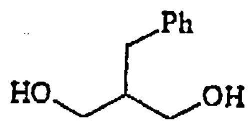 (VI)

by reduction of a malonic ester of the formula (V):

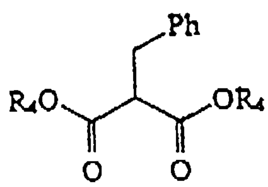 (V)

in which $R_4$ represents an alkyl chain containing from 1 to 4 carbon atoms, in the presence of a hydride;
b) preparing the monoacetate (VII) of (R) configuration or of (S) configurations, respectively, of the formula (VII R) or (VII S):

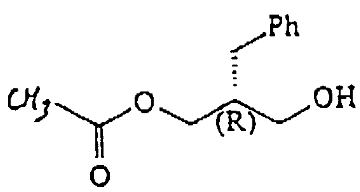

(VIIR)

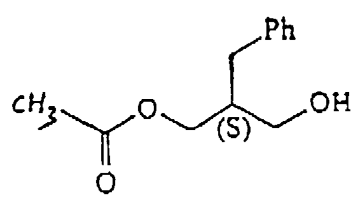

(VIIS)

c) subjecting the monoacetates of the formula (VII R) or (VII S) to an oxidation to form the acids of the formula (IX S) or (IX R):

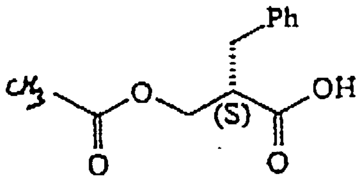

(IXS)

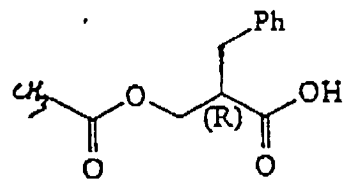

(IXR)

d) hydrolysing the compounds of the formula (IX S) or (IX R) in the presence of a basic aqueous solution to form the hydroxy acids of the formula (x S) or (X R) :

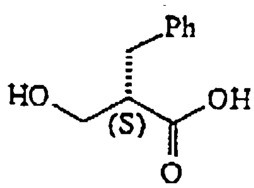

(XS)

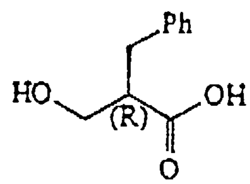

(XR)

e) thioacylating the hydroxy acids of the formula (X S) or (X R) with a mercapto acid of the formula (XI) :

$$R_1SH \quad (XI)$$

in which $R_1$ represents a linear or branched aliphatic acyl radical or an aromatic acyl radical, according to a Mitsunobu-type reaction in the presence of an alkyl azodicarboxylate/triphenylphosphine complex to give, respectively, the desired acids of the formula (I R) or (I S):

(I S):

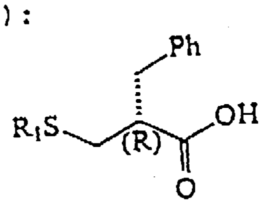

(IR)

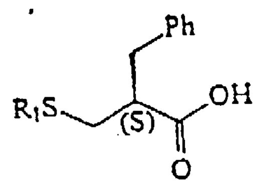

(IS)

**2.** Process according to Claim 1, characterized in that the monoacetate (VII R) of (R) configuration is obtained by reacting the diol of the formula (VI) with vinyl acetate in the presence of an enzyme.

**3.** Process according to Claim that 1, characterized in that the monoacetate (VII S) of (S) configuration is obtained by preparing the diacetate of the formula (VIII) :

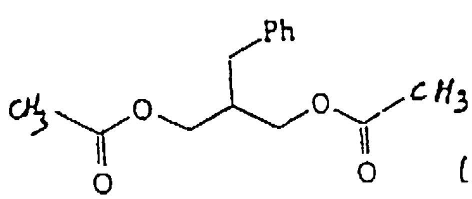

(VIII)

from the diol of the formula (VI), followed by enantioselective monohydrolysis of the diacetate of the formula (VIII) in the presence of an enzyme to form the monoacetate derivative.

4. Process according to Claim 3, characterized in that the diacetate of the formula (VIII) is obtained by reacting the diol of the formula (VI) with acetic anhydride in the presence of a catalyst.

5. Process according to Claim 3, characterized in that the diacetate of the formula (VIII) is obtained by reacting the diol of the formula (VI) with vinyl acetate in the presence of an enzyme.

6. Process according to any one of Claims 1 to 5, characterized in that the hydride used in step a) is chosen from lithium aluminium hydride and sodium borohydride.

7. Process according to Claim 2, characterized in that the enzyme used in step b) for the preparation of the monoacetate (VII R) is chosen from lipase PS (Amano) and the lipase obtained from Pseudomonas fluorescens (Fluka).

8. Process according to either of Claims 3 and 4, characterized in that the catalyst used in step b) for the diacetylation of the diol (VI) is chosen from the 4-dimethylaminopyridine/triethylamine mixture and sulphuric acid.

9. Process according to either of Claims 3 and 5, characterized in that the enzyme used in step b) for the diacetylation reaction of the diol (VI) is Novozym 435 (Novo Nordisk).

10. Process according to Claim 3, characterized in that the enzyme used in step b) for the monohydrolysis of the diacetate (VIII) is chosen from the lipase obtained from Pseudomonas fluorescens (Fluka) and lipase PS (Amano).

11. Process according to one of the preceding Claims, characterized in that the oxidizing agent used in step c) is chosen from Jones' reagent, potassium permanganate and nitric acid.

12. Process according to any one of the preceding claims, characterized in that the basic aqueous solution used in step d) is chosen from aqueous lithium hydroxide solution and aqueous sodium hydroxide solution.

13. Process according to any one of the preceding claims, characterized in that the alkyl azodicarboxylate used in step e) for the Mitsunobu reaction is chosen from diisopropyl azodicarboxylate and diethyl azodicarboxylate.

14. Process according to any one of the preceding claims, characterized in that the mercapto acid of the formula (XI) used in step e) for the Mitsunobu reaction is chosen from thioacetic acid and thiobenzoic acid.

15. Process according to any one of the preceding claims, characterized in that, when it is applied to the preparation of (R)-2-acetylthiomethyl-3-phenylpropanoic acid ($R_1$ = acetyl) of the formula (Ia R):

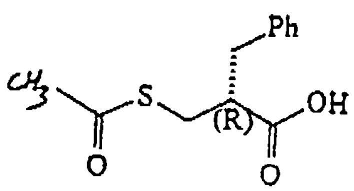
(IaR)

it comprises the steps of

a) carrying out a reduction of a malonic ester, such as dimethyl benzylmalonate ($R_4 = CH_3$), using a hydride, such as lithium aluminium hydride, in a solvent, such as tetrahydrofuran, to give the corresponding diol (VI):
b) carrying out a monoacetylation of the resultant diol of the formula (VI) in vinyl acetate in the presence of lipase PS (Amano),
c) oxidizing the alcohol function of the resultant monoacetate of the formula (VII R) in acetone using Jones' reagent ($CrO_3$- $H_2SO_4$),
d) hydrolysing the acetate function with an aqueous alkaline solution, such as aqueous lithium hydroxide solution, followed by releasing, by acidification, the hydroxy acid of the formula (X S):

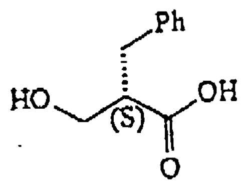

(XS)

e) substituting the alcohol function of the hydroxy acid of the formula (X S) according to a Mitsunobu-type reaction in the presence of triphenylphosphine/diisopropyl azodicarboxylate complex and thioacetic acid, to give the acid of (R) configuration of the formula (Ia R).

**16.** Process according to any one of Claims 1 to 14, characterized in that, when it is applied to the preparation of (S)-2-acetylthiomethyl-3-phenylpropanoic acid of the formula (Ia S):

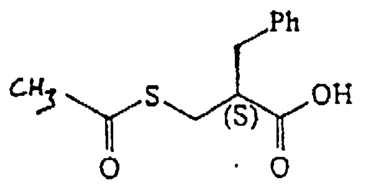

(IaS)

it comprises the steps of

a) carrying out a reduction of a malonate (V), such as dimethyl benzylmalonate ($R_4 = CH_3$), using a hydride, such as lithium aluminium hydride,

b) carrying out a diacetylation of the diol of the formula (VI) in vinyl acetate in the presence of Novozym 435 enzyme (Novo Nordisk), followed by carrying out a monohydrolysis of the resultant diacetate of the formula (VIII) in pH 7 phosphate buffer in the presence of lipase from Pseudomonas fluorescens (Fluka),

c) oxidizing the alcohol function of the monoacetate of the formula (VII S) in acetone using Jones' reagent ($CrO_3$ - $H_2SO_4$),

d) hydrolysing the acetate function with an aqueous alkaline solution, such as aqueous lithium hydroxide solution, followed by releasing, by acidification, the hydroxy acid of the formula (X R):

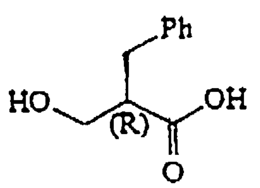

(XR)

e) substituting the alcohol function of the hydroxy acid of the formula (X R) according to a Mitsunobu-type reaction in the presence of a triphenylphosphine/diisopropyl azodicarboxylate complex and thioacetic acid, to give the acid of (S) configuration of the formula (Ia S).

**17.** Process for the preparation of optically active N-(mercaptoacyl)amino acid derivatives of the formula (II):

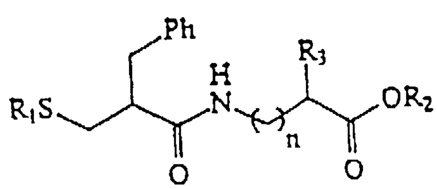

(II)

in which:

$R_1$ represents a hydrogen atom, a linear or branched aliphatic acyl radical or an aromatic acyl radical,
$R_2$ represents a hydrogen atom, a lower alkyl radical, a phenyl radical or a lower phenylalkylene group;
$R_3$ represents a hydrogen atom; a lower alkyl group; a lower hydroxyalkylene group; a phenyl group; a lower phenylalkylene group; a lower hydroxyphenylalkylene group; a lower aminoalkylene group; a lower guanidinoalkylene group; a lower mercaptoalkylene group; a lower alkyl lower thioalkylene group; a lower imidazolylalkylene group; a lower indolylalkylene group; a lower carbamylalkylene group; a lower carboxyalkylene group;
n ranges from 0 to 10;

characterized in that it comprises using the S-acyl derivatives of 2-mercaptomethyl-3-phenylpropanoic acid of the formula (I) in optically pure form, after they have been obtained using the process of Claims 1 to 16.

**18.** Process according to Claim 17, characterized in that it comprises using the compound of the formula (Ia R) or (Ia S).

**19.** Process according to Claim 17, characterized in that the compound (Ia R) is used for the synthesis of the compound of the formula (III) :

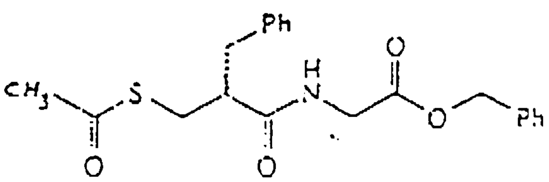
(III)

**20.** Process according to Claim 17, characterized in that the compound (Ia S) is used for the synthesis of the compound of the formula (IV):

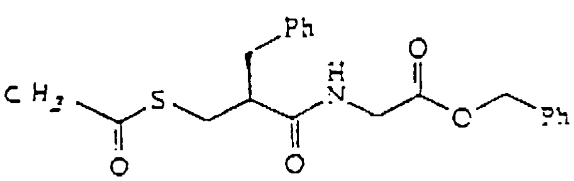
(IV)

**21.** Process according to any one of Claims 17 to 20, characterized in that it comprises the steps of:

f) forming the chloride (XII S) or (XII R)

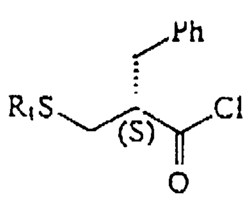
(XIIS)

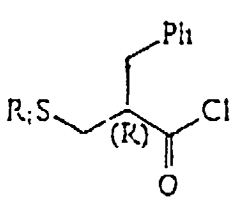
(XIIR)

of the acid (I R) or (I S) using a chlorinating agent, and
g) reacting the acid chloride (XII S) or (XII R) with an amino ester of the formula (XIII)

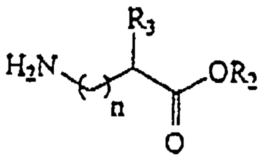

(XIII)

in the presence of a base.

**22.** Use of the S-acyl derivatives of optically pure 2-mercaptomethyl-3-phenylpropanoic acid of the formula (I) obtained by the process according to any one of Claims 1 to 16, for the synthesis of optically pure N-(mercaptoacyl)amino acid derivatives of the formula (II):

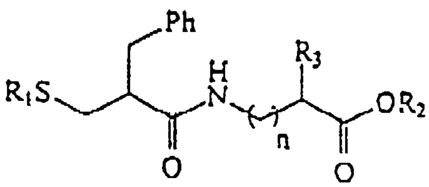

(II)

in which:

$R_1$ represents a hydrogen atom, a linear or branched aliphatic acyl radical or an aromatic acyl radical,
$R_2$ represents a hydrogen atom, a lower alkyl radical, a phenyl radical or a lower phenylalkylene group;
$R_3$ represents a hydrogen atom; a lower alkyl group; a lower hydroxyalkylene group; a phenyl group; a lower phenylalkylene group; a lower hydroxyphenylalkylene group; a lower aminoalkylene group; a lower guanidinoalkylene group; a lower mercaptoalkylene group; a lower alkyl lower thioalkylene group; a lower imidazolylalkylene group; a lower indolylalkylene group; a carbamylalkylene group; a lower carboxyalkylene group;
n ranges from 0 to 10.

**23.** Use, according to Claim 22, of (R)-2-acetylthiomethyl-3-phenylpropanoic acid of the formula (Ia R) for the synthesis of the derivatives of the formula (II).

**24.** Use, according to Claim 23, of (R)-2-acetylthiomethyl-3-phenylpropanoic acid of the formula (Ia R) for the synthesis of benzyl N-(R)-[2-acetylthiomethyl-1-oxo-3-phenylpropyl]glycinate of the formula (III).

**25.** Use, according to Claim 22, of (S)-2-acetylthiomethyl-3-phenylpropanoic acid of the formula (Ia S) for the synthesis of the derivatives of the formula (II).

**26.** Use, according to Claim 25, of (S)-2-acetylthiomethyl-3-phenylpropanoic acid of the formula (Ia S) for the synthesis of benzyl N-(S)-[2-acetylthiomethyl-1-oxo-3-phenylpropyl]glycinate of the formula (IV).